# EUROPEAN PATENT APPLICATION

(11) **EP 4 730 343 A1**
(43) Date of publication of application: **22.04.2026**
(21) Application number: 23941117.6
(22) Date of filing: 16.06.2023
(51) Int. Cl.: G16B 50/00

(54) **METHOD AND APPARATUS FOR DNA STORAGE ENCODING/DECODING AND RULES THEREOF**

(71) Applicant: BGI Research Shenzhen, Yantian District Shenzhen, Guangdong 518083 (CN)
(72) Inventor: ZHANG, Haoling, Shenzhen, Guangdong 518083 (CN); PING, Zhi, Shenzhen, Guangdong 518083 (CN); YAN, Xu, Shenzhen, Guangdong 518083 (CN); SHEN, Yue, Shenzhen, Guangdong 518083 (CN)
(74) Representative: Meissner Bolte Nürnberg
(86) International application number: PCT/CN2023/100857
(87) International publication number: WO 2024/254884

(57) **Abstract**

Disclosed are a method and apparatus for DNA storage encoding/decoding and rules thereof. The method comprises: executing single-molecule sequencing on a reference sequence, and acquiring actual sequencing data of the single-molecule sequencing; comparing the actual sequencing data with reference data of the reference sequence, counting the frequency, in the actual sequencing data, of sequencing errors of each sequence segment having a length of k, and calculating the proportion, in the actual sequencing data, of sequencing errors of each sequence segment having a length of k, that is, the error rate; and performing removal by using a sequence segment, the error rate of which exceeds a threshold, as a limiting condition. In the method of the present invention, the steps of DNA storage encoding/decoding are simplified, the time sequence of the steps is eliminated by using a threshold, and the complexity of data processing is reduced.

## Description

### TECHNICAL FIELD

The present disclosure relates to the field of biotechnology, and in particular to a method and apparatus for DNA storage encoding/decoding and for generating rule thereof.

### BACKGROUND OF THE INVENTION

With the development of modern technology, global data is increasing at an exponential rate. The growing volume of data places increasingly higher demands on storage technologies. Traditional storage technologies, such as tapes and optical discs, are becoming increasingly unable to meet current data needs due to their low storage density and limited lifespan. In recent years, the emerging DNA storage technology has provided a new solution to these problems. Compared with the traditional storage media, DNA, as a medium for information storage, has the characteristics of long lifespan (up to thousands of years or more, which is over 100 times longer than that of existing tape and optical disc media), high storage density (up to about 10⁹ Gb/mm³, which is over 10 million times higher than that of existing tape and optical disc media), high storage security and the like.

A general workflow of DNA data storage involves the following steps: firstly, binary information is converted into DNA sequences by a specific information conversion process; secondly, DNA molecules are synthesized chemically or enzymatically to physically store the corresponding DNA sequence information; and finally, the DNA molecules are sequenced, and the information is read by using the aforementioned information conversion process. The information conversion process, as the starting and ending points of the DNA storage workflow, plays a crucial role in the effective conversion between binary information and DNA sequences.

There is an important drawback in the DNA data storage that the process from raw sequencing data to decoding of information *(i.e.,* the final step as mentioned above) is very time-consuming and involves numerous steps. After obtaining raw sequencing data, there are generally 5 data processing steps as follows, to obtain data information: (1) clustering: preliminarily filtered DNA sequences are classified by using a specific clustering method; (2) alignment: multiple DNA sequences in each class are aligned to determine the base with a highest probability at each position in the sequences, and a sequence composed of the bases with the highest probability at different positions is called a high-confidence DNA sequence; (3) decoding: the resultant high-confidence DNA sequence is decoded to obtain a corresponding bit sequence, and DNA sequences that fail to be decoded are discarded; (4) error correction: the decoded bit sequence is corrected according to the error correction regions (comprising RS codes or other error correction codes) in the sequence, the bit sequences that cannot be corrected are eliminated, and after error correction, the error correction regions are removed; and (5) assembly: the corrected bit sequences are sorted according to the index regions of the bit sequences, so as to obtain data information.

The above five steps have a sequential nature and are carried out in the order of steps. However, this obviously leads to a delay in the retrieval process of DNA storage, thereby exacerbating the drawbacks of DNA data storage. Moreover, single-molecule sequencing has increasingly gained favor in the industry of DNA data storage. This technology has an advantage of fast sequencing speed, but its high error rate is one of the important bottlenecks restricting its wide application. The high error rate will render the clustering, alignment and error correction in the traditional retrieval process inapplicable. To combine decoding and error correction into one step, William H. Press, an academician from the National Academy of Sciences, proposed HEDGES in 2020, which uses a hash function and greedy exhaustive search to correct most index and substitution errors (Press W H, Hawkins J A, Jones Jr S K, et al. HEDGES error-correcting code for DNA storage corrects indels and allows sequence constraints[J]. Proceedings of the National Academy of Sciences, 2020, 117(31): 18489-18496.). This method can correct errors in single-molecule sequencing with a certain probability. However, a problem caused by the principle of this method *(i.e.,* greedy exhaustive search) is relatively slow speed (about 1 bit/second).

In view of the above, it is necessary to develop a retrieval process or mechanism that can perform real-time decoding of raw sequencing data and is resistant to high error rates (for example, in single-molecule sequencing).

### SUMMARY OF THE INVENTION

The objective of the present disclosure, at least partially, lies in addressing the problem of repairing DNA sequences with high error rates caused by single-molecule sequencing in DNA data storage, and in achieving end-to-end rapid retrieval to reach a decoding throughput at the GB level per sequence.

According to a first aspect of the present disclosure, the present disclosure provides a method for DNA storage encoding/decoding of single-molecule sequencing, comprising:
1) setting a sliding window (k, n) of DNA storage encoding/decoding rule, wherein k represents the length of sliding window, and n represents the length of base characters of each sliding, and wherein n and k are positive integers, k ≥ n;
2) obtaining the universal set of sequences with length k of sliding window, wherein the universal set of sequences is the collection of all nucleotide sequences that are formed by the random combination of all possible bases at each base position within the range of the sliding window length;
3) performing single-molecule sequencing on a reference sequence to obtain actual sequencing data of single-molecule sequencing;
4) aligning the actual sequencing data with the reference data of the reference sequence, counting the frequency of sequencing errors for each sequence fragment with the length of k in the actual sequencing data, and calculating the proportion of sequencing errors *(i.e.,* error rate) for each sequence fragment with the length of k in the actual sequencing data;
5) by imposing a constraint that removes sequence fragments with error rates above a threshold, screening for a set of qualified sequences with an error rate not exceeding the threshold from the universal set of sequences;
6) connecting the sequences in the set of qualified sequences by means of a directed graph, wherein each node in the directed graph represents each sequence; and
7) obtaining an algorithm diagram, wherein the algorithm diagram comprises DNA storage encoding/decoding rule.

In a preferred embodiment, the single-molecule sequencing comprises Nanopore single-molecule sequencing and/or other single-molecule sequencing technologies.

In a preferred embodiment, after step 6), the method further comprises:
i) deleting, in the directed graph, the nodes of which the number of out-degrees is less than a set limiting value for number of out-degrees; and
ii) deleting excessive out-degrees of each node in the directed graph, wherein the excessive out-degrees are those exceeding the set limiting value for number of out-degrees.

In a preferred embodiment, the step of deleting excessive out-degrees of each node in the directed graph comprises: if a total number of the out-degrees of the node exceeds the set limiting value for the number of out-degrees, the bases of the node are output in reverse order, and the out-degrees directed to the corresponding bases are deleted sequentially according to the order of bases output in reverse order.

In a preferred embodiment, collaborative sampling of error rates and sequence features of the sequencing data is performed with the set sliding window (k, n) as an observation scale of the actual sequencing data; and the error proportion of the sequencing data is statistically sorted according to the principle that higher error rates are given higher priority.

In a preferred embodiment, the error rate ranges from 2% to 4.5%, and preferably, the error rate is 3%.

According to a second aspect of the present disclosure, the present disclosure provides an apparatus for generating DNA storage encoding/decoding rule, comprising:
a unit for setting sliding window, configured to set a sliding window (k, n) of DNA storage encoding/decoding rule, wherein k represents the length of sliding window, and n represents the length of base characters of each sliding, and wherein n and k are positive integers, k ≥ n;
a unit for determining constraint, configured to perform single-molecule sequencing on a reference sequence to obtain the actual sequencing data of the single-molecule sequencing; to align the actual sequencing data with the reference data of the reference sequence, count the frequency of sequencing errors for each sequence fragment with the length of k in the actual sequencing data, and calculate the proportion of sequencing errors *(i.e.,* error rate) for each sequence fragment with the length of k in the actual sequencing data; and to impose a constraint of sequence fragments with error rates above a threshold;
a unit for screening qualified sequences, configured to obtain the universal set of sequences with length k of sliding window, wherein the universal set of sequences is the collection of all nucleotide sequences that are formed by the random combination of all possible bases at each base position within the range of the sliding window length; and to screen a set of qualified sequences that pass the constraint from the universal set of sequences;
a unit for connecting directed graph, configured to connect the sequences in the set of the qualified sequences by means of a directed graph, wherein each node in the directed graph represents each sequence; and
a unit for obtaining algorithm diagram, configured to obtain an algorithm diagram, wherein the algorithm diagram comprises DNA storage encoding/decoding rule.

In a preferred embodiment, the apparatus for generating a DNA storage encoding/decoding rule further comprises:
a unit for deleting unqualified nodes, configured to delete, in the directed graph, the nodes of which the number of out-degrees is less than a set limiting value for number of out-degrees; and
a unit for deleting excessive out-degree, configured to delete excessive out-degrees of each node in the directed graph, wherein the excessive out-degrees are those exceeding the set limiting value for number of out-degrees.

According to a third aspect of the present disclosure, the present disclosure provides a DNA storage encoding method, comprising:
1) obtaining the DNA storage encoding/decoding rule generated by the method according to the first aspect, and setting an initial node as the current node;
2) obtaining a binary sequence to be encoded and slicing it to generate binary slices, and converting the binary values corresponding to the slices into the out-degree node or the multi-layer out-degree node connected with the current node, wherein each out-degree node denotes a nucleic acid fragment and the binary slices form a one-to-one mapping relationship with the corresponding nucleic acid fragments;
3) according to the DNA storage encoding/decoding rule, inputting the binary slices to output the nucleic acid fragment that the out-degree node or the multi-layer out-degree node is mapped to, updating the out-degree node as the current node, and cyclically inputting the binary slices and outputting the nucleic acid fragments according to the order of the binary slices, until all of the binary slices have been input; and
4) connecting the nucleic acid fragments in the output order and outputting the complete DNA sequence.

In a preferred embodiment, in the method, the binary sequence to be encoded is sliced with the length of 2n-1, wherein n represents the length of base characters of each sliding of the sliding windows.

In a preferred embodiment, the method further comprises: synthesizing the DNA sequence and preserving it in an *ex vivo* medium or in living cells.

According to a fourth aspect of the present disclosure, the present disclosure provides a DNA storage encoding apparatus, comprising:
a unit for acquiring encoding/decoding rule, configured to obtain the DNA storage encoding/decoding rule generated by the method according to the first aspect, and set an initial node as the current node;
a unit for slicing and converting binary sequences, configured to obtain a binary sequence to be encoded and slice it to generate binary slices, and convert the binary values corresponding to the slices into the out-degree node or the multi-layer out-degree node connected with the current node, wherein each out-degree node denotes a nucleic acid fragment and the binary slices form a one-to-one mapping relationship with the corresponding nucleic acid fragments;
a unit for outputting nucleic acid fragment, configured to, according to the DNA storage encoding/decoding rule, input the binary slices to output the nucleic acid fragment that the out-degree node or the multi-layer out-degree node is mapped to, update the out-degree node as the current node, and cyclically input the binary slices and output the nucleic acid fragments, according to the order of the binary slices, until all of the binary slices have been input; and
a unit for connecting nucleic acid fragments, configured to connect the nucleic acid fragments in the output order and output the complete DNA sequence.

In a preferred embodiment, the binary sequence to be encoded is sliced with the length of 2n-1, wherein n represents the length of base characters of each sliding of the sliding windows.

According to a fifth aspect of the present disclosure, the present disclosure provides a DNA storage decoding method, comprising:
1) obtaining the DNA storage encoding/decoding rule generated by the method according to the first aspect, and setting an initial node as the current node;
2) obtaining a single-molecule sequenced DNA sequence to be decoded and slicing it to generate nucleic acid slices, and determining the out-degree node or the multi-layer out-degree node connected with the current node according to the DNA storage encoding/decoding rule and the nucleic acid information corresponding to the slices, wherein each out-degree node denotes the information of a nucleic acid fragment and the nucleic acid slices form a one-to-one mapping relationship with the corresponding binary values or binary slices;
3) for the current node and the out-degree node or the multi-layer out-degree node, obtaining the binary value or the binary slice between the nodes according to the mapping, updating the out-degree node as the current node, and cyclically inputting the nucleic acid slices and outputting the binary values or the binary slices, according to the order of the nucleic acid slices, until all of the nucleic acid slices have been input; and
4) connecting the binary values or the binary slices in the output order and outputting the complete binary sequence.

In a preferred embodiment, step 3) comprises an error correction step that uses a path-based probabilistic error correction strategy, comprising:
(1) detecting errors during decoding:
   detecting an error according to the constraint, that is, the current node has no access to the next node or does not meet the requirements of a valid path;
(2) determining the position where the error occurs during decoding:
   checking in reverse order, *i.e.,* backwards from the position where the error occurs to the position that is the length of an observation window away from the error position;
(3) obtaining candidate repaired sequences:
   after finding the position where the error occurs during decoding, performing a local exhaustive backward search to determine the error type and attempt to repair by the following three types of adjustments: replacing the nucleotide at the current position with another nucleotide, inserting a nucleotide between the current position and the previous position, and deleting the nucleotide at the current position, so as to obtain candidate repaired sequences; and
(4) obtaining a set of repaired DNA sequences:
   for the candidate repaired sequences, performing path detection based on Varshamov-Tenengolts error correction code to collect the candidate repaired sequences that pass the constraint and meet the requirements of the detected sequence; and outputting a set of repaired DNA sequences that pass the constraint and meet the requirements of the detected sequence.

In a preferred embodiment, after step (4), all of the sequences in the set of DNA sequences are counted and sorted, and the sequences with a frequency reaching a threshold are correct sequences, and are directly decoded.

In a preferred embodiment, the sequencing depth of the sequences is 20×, and the sequence frequency threshold is 4, that is, the sequences with a frequency reaching 4 are correct sequences, and are directly decoded.

In a preferred embodiment, in the method, the DNA sequence to be decoded is sliced with the length of n, wherein n represents the length of base characters of each sliding of the sliding windows.

In a preferred embodiment, the DNA sequence to be decoded is generated by being encoded by the method according to the third aspect or the apparatus according to the fourth aspect.

According to a sixth aspect of the present disclosure, the present disclosure provides a DNA storage decoding apparatus, comprising:
a unit for acquiring encoding/decoding rule, configured to obtain the DNA storage encoding/decoding rule generated by the method according to the first aspect, and set an initial node as the current node;
a unit for slicing and converting DNA, configured to obtain a DNA sequence to be decoded and slice it to generate nucleic acid slices, and determine the out-degree node or the multi-layer out-degree node connected with the current node according to the DNA storage encoding/decoding rule and the nucleic acid information corresponding to the slices, wherein each out-degree node denotes the information of a nucleic acid fragment and the nucleic acid slices form a one-to-one mapping relationship with the corresponding binary values or binary slices;
a unit for outputting binary value, configured to, for the current node and the out-degree node or the multi-layer out-degree node, obtain the binary value or the binary slice between the nodes according to the mapping, update the out-degree node as the current node, and cyclically input the nucleic acid slices and output the binary values or the binary slices, according to the order of the nucleic acid slices, until all of the nucleic acid slices have been input; and
a unit for connecting binary value, configured to connect the binary values in the output order and output the complete binary sequence.

In a preferred embodiment, the DNA sequence to be decoded is sliced with the length of n, wherein n represents the length of base characters of each sliding of the sliding windows.

According to a seventh aspect of the present disclosure, the present disclosure provides a computer-readable storage medium, comprising a program, wherein the program can be executed by a processor to achieve, for example, the method according to the first aspect or the method according to the third aspect or the method according to the fifth aspect.

The present disclosure designs a real-time repair mechanism without any preprocessing and strict order (for 2% of editing errors, the repair speed can reach 12,000 bits/second). The method of the present disclosure simplifies the steps of DNA storage encoding/decoding, eliminates the sequential nature of the steps by using a threshold, and reduces the complexity of data processing.

### BRIEF DESCRIPTION OF THE DRAWINGS

To illustrate the technical solutions of the embodiments of the present disclosure more clearly, the drawings in the embodiments are briefly described as below.

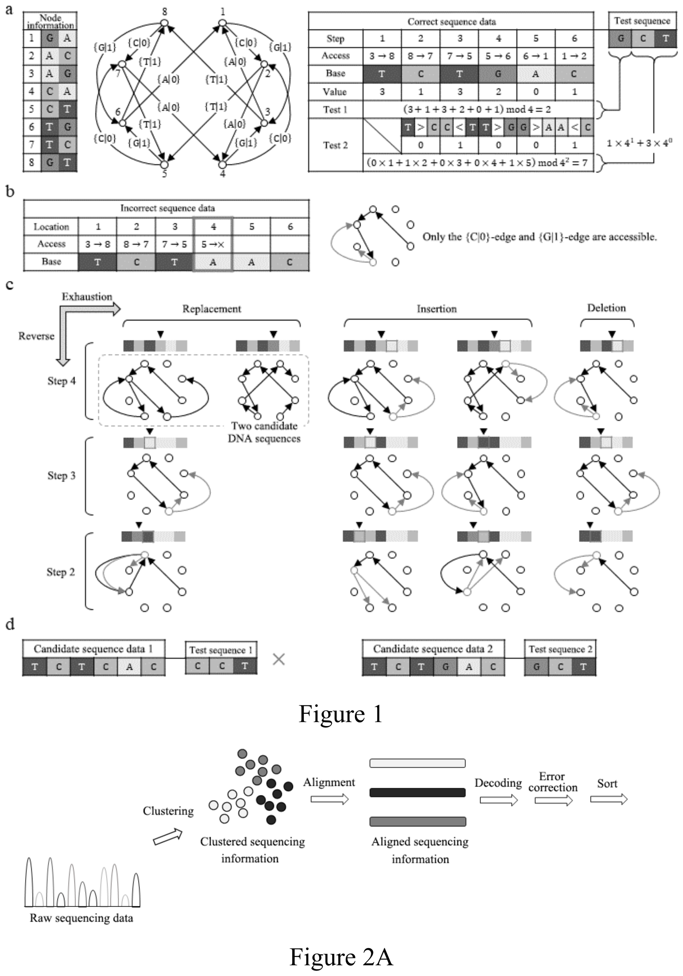

Figure 1 shows the path-based error correction strategy of the present disclosure. In (a), a GC-balanced directed graph with a length of 2 is created; by means of this directed graph, the binary message of 001101 is encoded into the DNA sequence TCTGAC; and a VT-based test string of GCT is generated from this DNA sequence. In (b), a real-time error detection during decoding is shown. In (c), a process of local exhaustive reverse search is shown; and two candidate strings are obtained by 13 searches. In (d) a final check process using the VT-based check is shown; and only one of the two candidates corresponds to the correct check value, finally resulting in the only solution.
Figure 2A shows an end-to-end retrieval mechanism in the prior art.
Figure 2B shows the end-to-end retrieval mechanism according to the present disclosure, which involves one less step compared to the strategy in Figure 2A, and simultaneously gets rid of strict asynchronous requirement. For example, if a sequence has a frequency of greater than 4, it can be decoded directly.
Figure 3 is a flowchart of the method for generating the DNA storage encoding/decoding rule in an example of the present disclosure.
Figure 4 is a flowchart of the DNA storage encoding method in an example of the present disclosure.
Figure 5 is a flowchart of the DNA storage decoding method in an example of the present disclosure.
Figure 6 shows an example image used in an example.

### DETAILED DESCRIPTION OF THE INVENTION

Hereinafter, the present disclosure will be described in detail by means of the embodiments in combination with the drawings. In the following embodiments, many details are described to facilitate a better understanding of the present disclosure. However, those skilled in the art can recognize without any effort that some features can be omitted or can be replaced by other materials and methods in different situations.

Furthermore, the characteristics, operations or features described in the description can be combined in any appropriate manner to form various embodiments. Additionally, the steps or actions described in the method can be reordered or adjusted in a way that is obvious to those skilled in the art. Therefore, the order in the description and drawings are merely for the purpose of clearly describing a particular embodiment, and do not imply a necessary order, unless the description states that a certain order must be followed.

The terms used in the present disclosure are explained as follows:
Error rate refers to the probability that a certain DNA sequence or fragment is changed to another sequence or fragment.

Collaborative sampling refers to performing statistics analysis according to a correct sequence and the aligned sequenced sequences.

VT error correction code, also known as Varshamov-Tenengolts, corrects a single insertion/deletion error by asymptotically optimal redundancy.

Path refers to a connected path between nodes in a generated algorithm graph as described in WO/2021/243605.

Threshold refers to a certain predetermined value that indicates the number of occurrences of a certain sequence after repair.

Encoding method refers to a mapping between binary and base. Generally, the traditional encoding method with a fixed rule can perform optimization of a plurality of steps to finally obtain a final mapping. In the present disclosure, the encoding method is achieved by an encoding/decoding rule. The encoding/decoding rule of the present disclosure is generated by a method for generating a DNA storage encoding/decoding rule of the present disclosure.

Generator, also called "method for generating a DNA storage encoding/decoding rule" in the present disclosure, obtains a certain potential mapping between binary and base by means of a method of graph theory according to different combination situations, thereby obtaining the encoding/decoding rule of the present disclosure.

Algorithm stability means that, for any input electronic file, a DNA sequence output by an algorithm can stably meet a constraint. Generally, in any case, flood-like input can be used to observe the stability of an algorithm in an extreme case.

End-to-end refers to a data recovery mechanism that does not require clustering and alignment processes. From raw data input to result output, from input end to output end, the mapping process forms a self-contained system.

Time complexity of an algorithm is a function which qualitatively describes the running time of the algorithm. It is a function of string length representing an input value of the algorithm. Time complexity is often expressed in the notation of "O", excluding the lower-order terms and the leading coefficient of the function. While used in this way, time complexity can be deemed as being asymptotic, that is, the case in which an input value approaches infinity is investigated.

In view of the sequence constraints of different sequencing or synthesis instruments, the present disclosure proposes an optimal encoding/decoding generator by using the constraints, *i.e.,* the method for generating a DNA storage encoding/decoding rule of the present disclosure. This generator (or method) can solve the problem that the existing fixed rules cannot completely avoid extreme GC or some special motifs. The special motifs herein refer to sequences that are difficult to analyze by using the fixed rules.

In addition, the encoding method generated by the generator does not involve a screening process, and there is no risk of being unable to accept all of the inputs. In addition, the encoding/decoding time complexity of the encoding method generated by the generator is O(n). Compared with most encoding/decoding methods that involve many optimization processes, the encoding/decoding method of the present disclosure is much faster and is more efficient for large-scale DNA storage transcoding in future.

The technical components of the present disclosure are described in detail as below. It should be understood that these descriptions are exemplary, and those skilled in the art can make many variations on the basis of the technical contents of the present disclosure.

During single-molecule sequencing, every time a base is read, it is added to the end of the sequence that has already been obtained, and the sequence is corrected by using a path-based probabilistic error correction strategy. This correction speed is much faster than the sequencing speed, and therefore the path-based probabilistic error correction strategy can be used for real-time decoding. With the frequency sorting mechanism mentioned in the present disclosure, the goal of real-time decoding can be ultimately achieved. In the present disclosure, the path-based probabilistic error correction strategy can be referred to in Figure 1 and Zhang H , Lan Z , Zhang W ,et al. SPIDER-WEB enables stable, repairable, and encryptible algorithms under arbitrary local biochemical constraints in DNA-based storage[J], 2022.DOI:10.48550/arXiv.2204.02855., https://arxiv.org/abs/2204.02855, which is hereby incorporated by reference in its entirety. Figure 1 shows the path-based error correction strategy of the present disclosure. In (a), a GC-balanced directed graph with a length of 2 is created; by means of this directed graph, the binary message 001101 is encoded into the DNA sequence TCTGAC; and a VT-based test string GCT is generated from this DNA sequence. In (b), a real-time error detection during decoding is shown. In (c) a process of local exhaustive reverse search is shown; and two candidate strings are obtained by 13 searches. In (d), a final check process using the VT-based check is shown; and only one of the two candidates corresponds to the correct check value, finally resulting in the only solution. Therefore, the path-based error correction strategy can comprise: (1) firstly, detecting errors according to constraints, that is, the current node cannot access a next node (or cannot meet the requirements of a valid path); (2) for a node where an error occurs, in reverse order, performing check and path correction on the previous positions from the position where the error occurs, until reaching the position that is the length of an observation window away from the error position; (3) path correction, comprising guessing the type of error (one of the editing errors) and attempting to adjust it to one of the following three types for repairing: replacing the nucleotide at the current position with another nucleotide, inserting a nucleotide between the current position and the previous position, and deleting the nucleotide at the current position; (4) for the repaired sequences, performing the path detection based on Varshamov-Tenengolts error correction code and collecting the sequences that meet the detection requirements; and (5) outputting a set of repaired DNA sequences that pass the constraints and meet the requirements of detected sequence.

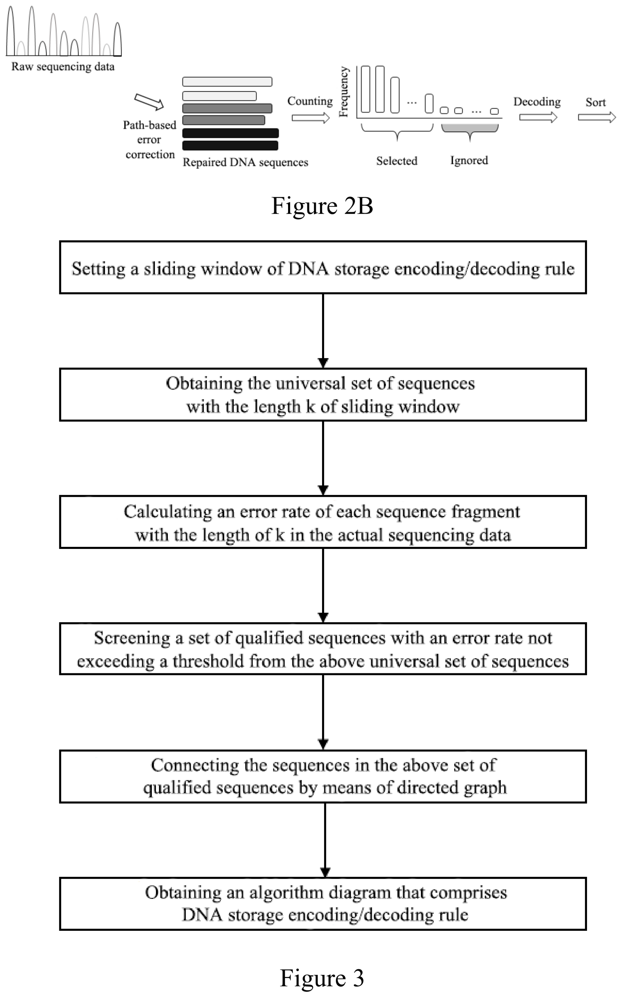

In the present disclosure, the frequency-based end-to-end retrieval mechanism can be referred to in Figures 2A and 2B. Figure 2A shows the existing end-to-end retrieval mechanism, and Figure 2B shows the end-to-end retrieval mechanism according to the present disclosure. Assuming that there are m types of the synthesized DNA sequences, (1) the path-based probabilistic error correction strategy is applied to each of the DNA sequences obtained by sequencing; (2) the set of repaired DNA sequences that pass the constraints and meet the requirements of detected sequence is count; (3) the DNA sequences are sorted according to the frequency priority principle; and (4) the first m sequences are taken as the final restored sequences and decoded. The frequency-based end-to-end retrieval mechanism of the present disclosure reduces the original five steps to four steps *(i.e.,* repairing (at DNA level) - counting - decoding - assembling). The correct sequences can be retrieved by frequency sorting according to the results of path error correction, without clustering and alignment. Meanwhile, the strategy of the present disclosure gets rid of the strict asynchronous requirements. For example, if a sequence has a frequency of greater than 4, it can be decoded directly. These steps can get rid of the necessary sequential nature by using a threshold. For example, when the sequencing depth is 20, as long as a certain sequence has a frequency of greater than 4, it can be directly regarded as a correct sequence for decoding and subsequent assembly. On the other hand, compared with a global search method, the path-based probabilistic error correction strategy of the present disclosure is a local search. The number of searches for nodes is reduced by an average of thousands of times (see Table 1), and the consumed time can theoretically be reduced by thousands of times. Table 1 shows the number of vertex accesses for local and global searches under different error rates. The DNA sequence has the length of 200 nt (that is, the minimum number of node accesses is 200 nt). The purpose of counting the number of search vertices rather than the actual consumed time is to eliminate the uncertain influence of implementation strategy differences and programming languages on the running time.

**Table 1:**

| Error Rate | Local Search | Global Search |
|---|---|---|
| 0.0% | 200.00(±0.00) | 13081139.25(±8302685.09) |
| 0.5% | 260.56(±7.79) | 13199144.94(±8120284.71) |
| 1.0% | 321.04(±11.02) | 13258923.94(±7935176.21) |
| 2.0% | 441.62(±15.81) | 14097832.26(±7135697.40) |
| 3.0% | 561.98(±19.14) | 15037965.54(±5963006.95) |

According to the first aspect of the present disclosure, the present disclosure provides a method for DNA storage encoding/decoding of single-molecule sequencing. As shown in Figure 3, an exemplary method for DNA storage encoding/decoding comprises: 1) setting a sliding window (k, n) of DNA storage encoding/decoding rule, wherein k represents the length of sliding window, and n represents the length of base characters of each sliding, and wherein n and k are positive integers, k ≥ n; 2) obtaining the universal set of sequences with length k of sliding window, wherein the universal set of sequences is the collection of all nucleotide sequences that are formed by the random combination of all possible bases at each base position within the range of the sliding window length; 3) performing single-molecule sequencing on a reference sequence to obtain actual sequencing data of single-molecule sequencing; 4) aligning the actual sequencing data with the reference data of the reference sequence, counting the frequency of sequencing errors for each sequence fragment with the length of k in the actual sequencing data, and calculating the proportion of sequencing errors *(i.e.,* error rate) for each sequence fragment with the length of k in the actual sequencing data; 5) by imposing a constraint that removes sequence fragments with error rates above a threshold, screening for a set of qualified sequences with an error rate not exceeding the threshold from the universal set of sequences; 6) connecting the sequences in the set of qualified sequences by means of a directed graph, wherein each node in the directed graph represents each sequence; and 7) obtaining an algorithm diagram, wherein the algorithm diagram comprises DNA storage encoding/decoding rule.

In the present disclosure, based on error sorting, the error-prone sequences are removed and a DNA storage encoding/decoding method is generated. The DNA storage encoding/decoding method can be referred to WO/2021/243605, which is hereby incorporated by reference in its entirety. Specifically, the error-prone sequence fragments need to be removed, and the sequence fragments with the calculated error rates less than or equal to 3% are used for DNA storage encoding/decoding. As referred to in WO/2021/243605, the DNA storage encoding/decoding is as follows: encoding by loading a graph of the algorithm and selecting an initial node; converting the binary sequence to decimal number, dividing the decimal number by the out-degrees of the current node, recording the subsequent DNA fragment information of the output out-degree node, updating the out-degree node as the current node, and performing the steps cyclically until there is no decimal number equal to 0; and connecting the DNA fragments in sequence and outputting the complete DNA sequence. Since the nodes accessed during encoding and decoding have the same path, the decoding is the mirror process of the encoding.

In the present disclosure, the steps of feature extraction and sorting strategy for errors of single-molecule sequencing can comprise: (1) setting a sliding window (k, n) of DNA storage encoding/decoding rule, wherein k represents the length of sliding window, and n represents the length of base characters of each sliding, and wherein n and k are positive integers, k ≥ n; 2) obtaining the universal set of sequences with length k of sliding window, wherein the universal set of sequences is the collection of all nucleotide sequences that are formed by the random combination of all possible bases at each base position within the range of the sliding window length; 3) performing single-molecule sequencing on a reference sequence to obtain actual sequencing data of single-molecule sequencing; and aligning the actual sequencing data with the reference data of the reference sequence, counting the frequency of sequencing errors for each sequence fragment with the length of k in the actual sequencing data, and calculating the proportion of sequencing errors *(i.e.,* error rate) for each sequence fragment with the length of k in the actual sequencing data; 4) statistically sorting the error rates of the sequencing data, and according to the principle that higher error rates are given higher priority, deleting the sequence fragments with an error rate exceeding a threshold to screen for a set of qualified sequences with an error rate not exceeding the threshold from the universal set of sequences.

The method of the present disclosure can be used to model single-molecule sequencing technology, including but not limited to ONT single-molecule sequencing, Nanopore single-molecule sequencing, etc. The single-molecule sequencing technology has a disadvantage of high error rate, and may cause problems when applied to DNA storage technology. The present disclosure improves the reliability of DNA storage technology by aligning sequencing data with a reference sequence to screen and remove the error-prone sequence fragments for DNA storage encoding. In the present disclosure, the reference sequence is used to correct the incorrect sequencing sequences in single-molecule sequencing data. Therefore, the reference sequence is as consistent as possible (preferably the same) with the DNA sample sequence of single-molecule sequencing. The reference sequence is a known sequence, which can be a genomic sequence, and is used to test the single-molecule sequencing system, so that the frequency and proportion of sequencing errors of each sequence fragment with the length of k in the actual sequencing data can be calculated. The reference sequence can be derived from publicly available genomic sequences or obtained by a sequencing technology.

In a preferred embodiment, after step 6), the method further comprises:
i) deleting, in the directed graph, the nodes of which the number of out-degrees is less than a set limiting value for number of out-degrees; and
ii) deleting excessive out-degrees of each node in the directed graph, wherein the excessive out-degrees are those exceeding the set limiting value for number of out-degrees.

In a preferred embodiment, the step of deleting excessive out-degrees of each node in the directed graph comprises: if a total number of the out-degrees of the node exceeds the set limiting value for the number of out-degrees, the bases of the node are output in reverse order, and the out-degrees directed to the corresponding bases are deleted sequentially according to the order of bases output in reverse order.

In a preferred embodiment, collaborative sampling of error rates and sequence features of the sequencing data is performed with the set sliding window (k, n) as an observation scale of the actual sequencing data; and the error proportion of the sequencing data is statistically sorted according to the principle that higher error rates are given higher priority.

In a preferred embodiment, the error rate ranges from 2% to 4.5%, and preferably, the error rate is 3%.

According to the second aspect of the present disclosure, the present disclosure provides an apparatus for generating DNA storage encoding/decoding rule, comprising:
a unit for setting sliding window, configured to set a sliding window (k, n) of DNA storage encoding/decoding rule, wherein k represents the length of sliding window, and n represents the length of base characters of each sliding, and wherein n and k are positive integers, k ≥ n;
a unit for determining constraint, configured to perform single-molecule sequencing on a reference sequence to obtain the actual sequencing data of the single-molecule sequencing; to align the actual sequencing data with the reference data of the reference sequence, count the frequency of sequencing errors for each sequence fragment with the length of k in the actual sequencing data, and calculate the proportion of sequencing errors *(i.e.,* error rate) for each sequence fragment with the length of k in the actual sequencing data; and to impose a constraint of sequence fragments with error rates above a threshold;
a unit for screening qualified sequences, configured to obtain the universal set of sequences with length k of sliding window, wherein the universal set of sequences is the collection of all nucleotide sequences that are formed by the random combination of all possible bases at each base position within the range of the sliding window length; and to screen a set of qualified sequences that pass the constraint from the universal set of sequences;
a unit for connecting directed graph, configured to connect the sequences in the set of the qualified sequences by means of a directed graph, wherein each node in the directed graph represents each sequence; and
a unit for obtaining algorithm diagram, configured to obtain an algorithm diagram, wherein the algorithm diagram comprises DNA storage encoding/decoding rule.

In a preferred embodiment, the apparatus for generating a DNA storage encoding/decoding rule further comprises:
a unit for deleting unqualified nodes, configured to delete, in the directed graph, the nodes of which the number of out-degrees is less than a set limiting value for number of out-degrees; and
a unit for deleting excessive out-degrees, configured to delete excessive out-degrees of each node in the directed graph, wherein the excessive out-degrees are those exceeding the set limiting value for number of out-degrees.

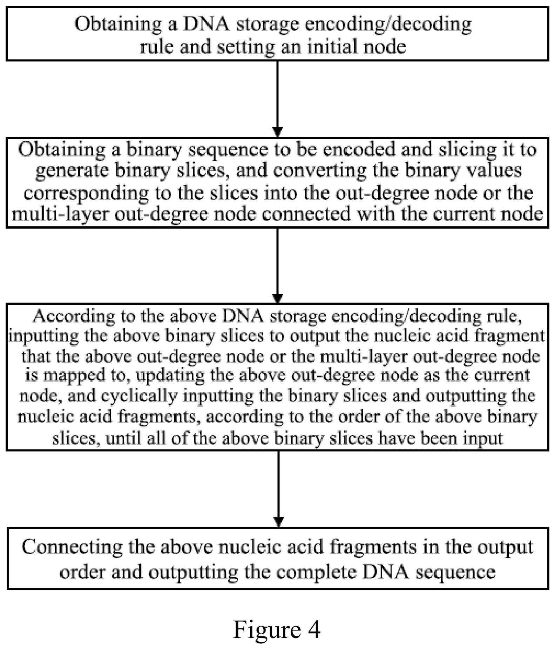

According to the third aspect of the present disclosure, the disclosure provides a DNA storage encoding method. As shown in Figure 4, an exemplary DNA storage encoding method comprises: 1) obtaining the DNA storage encoding/decoding rule generated by the method according to the first aspect, and setting an initial node as the current node; 2) obtaining a binary sequence to be encoded and slicing it to generate binary slices, and converting the binary values corresponding to the slices into the out-degree node or the multi-layer out-degree node connected with the current node, wherein each out-degree node denotes a nucleic acid fragment and the binary slices form a one-to-one mapping relationship with the corresponding nucleic acid fragments; 3) according to the DNA storage encoding/decoding rule, inputting the binary slices to output the nucleic acid fragment that the out-degree node or the multi-layer out-degree node is mapped to, updating the out-degree node as the current node, and cyclically inputting the binary slices and outputting the nucleic acid fragments, according to the order of the binary slices, until all of the binary slices have been input; and 4) connecting the nucleic acid fragments in the output order and outputting the complete DNA sequence.

In a preferred embodiment, in the method, the binary sequence to be encoded is sliced with the length of 2n-1, wherein n represents the length of base characters of each sliding of the sliding windows.

In a preferred embodiment, the method further comprises: synthesizing the DNA sequence and preserving it in an *ex vivo* medium or in living cells.

According to the fourth aspect of the present disclosure, the present disclosure provides a DNA storage encoding apparatus, comprising:
a unit for acquiring encoding/decoding rule, configured to obtain the DNA storage encoding/decoding rule generated by the method according to the first aspect, and set an initial node as the current node;
a unit for slicing and converting binary sequences, configured to obtain a binary sequence to be encoded and slice it to generate binary slices, and convert the binary values corresponding to the slices into the out-degree node or the multi-layer out-degree node connected with the current node, wherein each out-degree node denotes a nucleic acid fragment and the binary slices form a one-to-one mapping relationship with the corresponding nucleic acid fragments;
a unit for outputting nucleic acid fragment, configured to, according to the DNA storage encoding/decoding rule, input the binary slices to output the nucleic acid fragment that the out-degree node or the multi-layer out-degree node is mapped to, update the out-degree node as the current node, and cyclically input the binary slices and output the nucleic acid fragments, according to the order of the binary slices, until all of the binary slices have been input; and
a unit for connecting nucleic acid fragments, configured to connect the nucleic acid fragments in the output order and output the complete DNA sequence.

In a preferred embodiment, the binary sequence to be encoded is sliced with the length of 2n-1, wherein n represents the length of base characters of each sliding of the sliding windows.

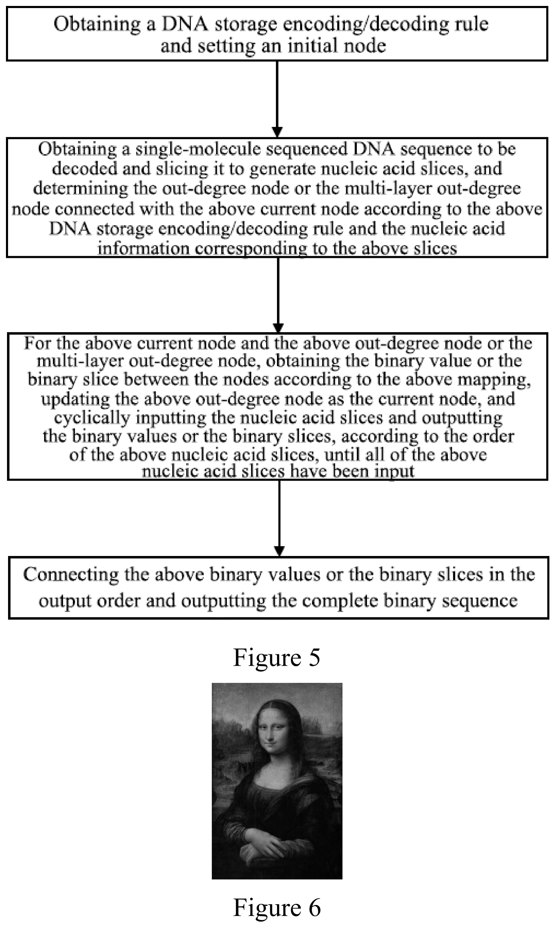

According to the fifth aspect of the present disclosure, the present disclosure provides a DNA storage decoding method. As shown in Figure 5, an exemplary DNA storage decoding method comprises: 1) obtaining the DNA storage encoding/decoding rule generated by the method according to the first aspect, and setting an initial node as the current node; 2) obtaining a single-molecule sequenced DNA sequence to be decoded and slicing it to generate nucleic acid slices, and determining the out-degree node or the multi-layer out-degree node connected with the current node according to the DNA storage encoding/decoding rule and the nucleic acid information corresponding to the slices, wherein each out-degree node denotes the information of a nucleic acid fragment and the nucleic acid slices form a one-to-one mapping relationship with the corresponding binary values or binary slices; 3) for the current node and the out-degree node or the multi-layer out-degree node, obtaining the binary value or the binary slice between the nodes according to the mapping, updating the out-degree node as the current node, and cyclically inputting the nucleic acid slices and outputting the binary values or the binary slices, according to the order of the nucleic acid slices, until all of the nucleic acid slices have been input; and 4) connecting the binary values or the binary slices in the output order and outputting the complete binary sequence.

In a preferred embodiment, step 3) comprises an error correction step that uses a path-based probabilistic error correction strategy, comprising: (1) detecting errors during decoding: detecting an error according to the constraint, that is, the current node has no access to the next node or does not meet the requirements of a valid path; (2) determining the position where the error occurs during decoding: checking in reverse order, *i.e.,* backwards from the position where the error occurs to the position that is the length of an observation window away from the error position; (3) obtaining candidate repaired sequences: after finding the position where the error occurs during decoding, performing a local exhaustive backward search to determine the error type and attempt to repair by the following three types of adjustments: replacing the nucleotide at the current position with another nucleotide, inserting a nucleotide between the current position and the previous position, and deleting the nucleotide at the current position, so as to obtain candidate repaired sequences; and (4) obtaining a set of repaired DNA sequences: for the candidate repaired sequences, performing path detection based on Varshamov-Tenengolts error correction code to collect the candidate repaired sequences that pass the constraint and meet the requirements of the detected sequence; and outputting a set of repaired DNA sequences that pass the constraint and meet the requirements of the detected sequence.

In a preferred embodiment, after step (4), all of the sequences in the set of DNA sequences are counted and sorted, and the sequences with a frequency reaching a threshold are correct sequences, and are directly decoded.

In a preferred embodiment, the sequencing depth of the sequences is 20×, and the sequence frequency threshold is 4, that is, the sequences with a frequency reaching 4 are correct sequences, and are directly decoded.

In a preferred embodiment, in the method, the DNA sequence to be decoded is sliced with the length of n, wherein n represents the length of base characters of each sliding of the sliding windows.

In a preferred embodiment, the DNA sequence to be decoded is generated by being encoded by the method according to the third aspect or the apparatus according to the fourth aspect.

According to the sixth aspect of the present disclosure, the present disclosure provides a DNA storage decoding apparatus, comprising:
a unit for acquiring encoding/decoding rule, configured to obtain the DNA storage encoding/decoding rule generated by the method according to the first aspect, and set an initial node as the current node;
a unit for slicing and converting DNA, configured to obtain a DNA sequence to be decoded and slice it to generate nucleic acid slices, and determine the out-degree node or the multi-layer out-degree node connected with the current node according to the DNA storage encoding/decoding rule and the nucleic acid information corresponding to the slices, wherein each out-degree node denotes the information of a nucleic acid fragment and the nucleic acid slices form a one-to-one mapping relationship with the corresponding binary values or binary slices;
a unit for outputting binary value, configured to, for the current node and the out-degree node or the multi-layer out-degree node, obtain the binary value or the binary slice between the nodes according to the mapping, update the out-degree node as the current node, and cyclically input the nucleic acid slices and output the binary values or the binary slices, according to the order of the nucleic acid slices, until all of the nucleic acid slices have been input; and
a unit for connecting binary value, configured to connect the binary values in the output order and output the complete binary sequence.

In a preferred embodiment, the DNA sequence to be decoded is sliced with the length of n, wherein n represents the length of base characters of each sliding of the sliding windows.

According to the seventh aspect of the present disclosure, the present disclosure provides a computer-readable storage medium, comprising a program, wherein the program can be executed by a processor to achieve, for example, the method according to the first aspect or the method according to the third aspect or the method according to the fifth aspect.

### Example

In this example, a sliding window (k, n) was set to have a length k of 4 and a base character length n of each sliding of 1. The example image is the Mona Lisa (see Figure 6). The image has a size of 2283400 bytes (approximately 2.17 MB), with a dimension of 709 pixels (width) × 1073 pixels (height), and a bit depth of 24.
1) The error rates of different sequences were collected by feature extracting and sorting strategy:
(1) performing Nanopore single-molecule sequencing on *Escherichia coli,*
obtaining the actual sequencing data of single-molecule sequencing, comprising obtaining the sequencing data of Nanopore single-molecule sequencing on *Escherichia coli;*
(2) aligning the actual sequencing data with the reference data of a reference sequence;
(3) extracting the correct sequence fragments and corresponding sequence fragments thereof in the sequencing data based on sliding window;
(4) generating 256 different DNA fragments based on the length of sliding window;
(5) counting the number of correct sequence fragments, a, and the number of fragments with errors obtained by sequencing, b, and calculating the error rate as b/(a+b);
(6) sorting the fragments in the descending order of error rates, to obtain the sorting result of the sliding windows; and
(7) determining the fragments with a calculated error rate greater than or equal to 3% ( 44 in total, see Table 2, wherein the sequences with high error rates are statistically counted by k-mer), and selecting them into a set that meets the constraints.

**Table 2:**

| Fragments with an error rate greater than or equal to 3% | | | | |
|---|---|---|---|---|
| AAAA | AAAC | AAAT | AAGA | AAGC |
| AAGG | AAGT | AATA | AATT | ACGC |
| AGAA | AGCA | AGCG | AGGA | AGGC |
| AGGG | AGGT | CAAA | CAAG | CAAT |
| CAGC | CAGG | CCCC | CCCV | CGCG |
| CGGA | CGGC | CGGG | CTAA | CTGG |
| GAAA | GAAG | GGAA | GGAT | GGCG |
| GGGC | GGGG | TAAA | TAAT | TGGA |
| TGGC | TGGG | TGGT | TTTT | |

2) The corresponding encoding method diagram was generated by deleting the fragments with an error rate greater than or equal to 3%:
(1) generating a set of qualified sequences with an error rate not exceeding a threshold from the universal set of sequences, by deleting the fragments with an error rate greater than or equal to 3% from 256 DNA fragments; and
(2) creating an encoding method diagram by applying the sequences in the set of qualified sequences to the method as described in patent application WO/2021/243605.

3) The example image was encoded by using encoding method diagram:
(1) setting the sequence length of 200 nt and the verification sequence length of 5 nt; and
(2) obtaining 60891 DNA sequences from the example sequences.

4) Each of the 60891 encoded DNA sequences was set to have a random error rate of 2% (comprising substitution, insertion and deletion) and a sequencing depth of 20×, to complete real-time decoding:
(1) performing the path-based error correction strategy on each DNA sequence to obtain the corresponding sequence solution set, merging the solution sets of all sequences and counting the frequency of each sequence solution;
(2) prioritarily decoding the repaired sequences with a frequency greater than 4, by the frequency-based end-to-end detection mechanism;
(3) running for approximately 1562 seconds on Intel(R) Core(TM) i7-4710MQ CPU (single core) to obtain an overall repair rate of approximately 98.18% (59783/60891) (the proportion of correct bit sequences among total bit sequences); and
(4) decoding the sequence information of the repaired DNA sequences according to the encoding rule to obtain binary values, and restoring the binary information to obtain the example image.

## Claims

1. A method for DNA storage encoding/decoding, comprising:
1) setting a sliding window (k, n) of DNA storage encoding/decoding rule, wherein k represents the length of sliding window, and n represents the length of base characters of each sliding, and wherein n and k are positive integers, k ≥ n;
2) obtaining the universal set of sequences with length k of sliding window, wherein the universal set of sequences is the collection of all nucleotide sequences that are formed by the random combination of all possible bases at each base position within the range of the sliding window length;
3) sequencing a reference sequence to obtain actual sequencing data;
4) aligning the actual sequencing data with the reference data of the reference sequence, counting the frequency of sequencing errors for each sequence fragment with the length of k in the actual sequencing data, and calculating the proportion of sequencing errors *(i.e.,* error rate) for each sequence fragment with the length of k in the actual sequencing data;
5) by imposing a constraint that removes sequence fragments with error rates above a threshold, screening for a set of qualified sequences with an error rate not exceeding the threshold from the universal set of sequences;
6) connecting the sequences in the set of qualified sequences by means of a directed graph, wherein each node in the directed graph represents each sequence; and
7) obtaining an algorithm diagram, wherein the algorithm diagram comprises DNA storage encoding/decoding rule.

2. The method of claim 1, wherein the sequencing is single-molecule sequencing.

3. The method of claim 2, wherein the single-molecule sequencing comprises ONT single-molecule sequencing and/or Nanopore single-molecule sequencing.

4. The method of claim 1, after step 6), further comprising:
i) deleting, in the directed graph, the nodes of which the number of out-degrees is less than a set limiting value for number of out-degrees; and
ii) deleting excessive out-degrees of each node in the directed graph, wherein the excessive out-degrees are those exceeding the set limiting value for number of out-degrees.

5. The method of claim 4, wherein the step of deleting excessive out-degrees of each node in the directed graph comprises: if a total number of the out-degrees of the node exceeds the set limiting value for the number of out-degrees, the bases of the node are output in reverse order, and the out-degrees directed to the corresponding bases are deleted sequentially according to the order of bases output in reverse order.

6. The method of any one of claims 1-5, wherein collaborative sampling of error rates and sequence features of the sequencing data is performed with the set sliding window (k, n) as an observation scale of the actual sequencing data; and the error proportion of the sequencing data is statistically sorted according to the principle that higher error rates are given higher priority.

7. The method of any one of claims 1-6, wherein the error rate ranges from 2% to 4.5%.

8. The method of any one of claims 1-7, wherein the error rate is 3%.

9. An apparatus for generating DNA storage encoding/decoding rule, comprising:
a unit for setting sliding window, configured to set a sliding window (k, n) of DNA storage encoding/decoding rule, wherein k represents the length of sliding window, and n represents the length of base characters of each sliding, and wherein n and k are positive integers, k ≥ n;
a unit for determining constraint, configured to perform single-molecule sequencing on a reference sequence to obtain the actual sequencing data of the single-molecule sequencing; to align the actual sequencing data with the reference data of the reference sequence, count the frequency of sequencing errors for each sequence fragment with the length of k in the actual sequencing data, and calculate the proportion of sequencing errors *(i.e.,* error rate) for each sequence fragment with the length of k in the actual sequencing data; and to impose a constraint of sequence fragments with error rates above a threshold;
a unit for screening qualified sequences, configured to obtain the universal set of sequences with length k of sliding window, wherein the universal set of sequences is the collection of all nucleotide sequences that are formed by the random combination of all possible bases at each base position within the range of the sliding window length; and to screen a set of qualified sequences that pass the constraint from the universal set of sequences;
a unit for connecting directed graph, configured to connect the sequences in the set of the qualified sequences by means of a directed graph, wherein each node in the directed graph represents each sequence; and
a unit for obtaining algorithm diagram, configured to obtain an algorithm diagram, wherein the algorithm diagram comprises DNA storage encoding/decoding rule.

10. The apparatus of claim 9, wherein the apparatus for generating a DNA storage encoding/decoding rule further comprises:
a unit for deleting unqualified nodes, configured to delete, in the directed graph, the nodes of which the number of out-degrees is less than a set limiting value for number of out-degrees; and
a unit for deleting excessive out-degrees, configured to delete excessive out-degrees of each node in the directed graph, wherein the excessive out-degrees are those exceeding the set limiting value for number of out-degrees.

11. A DNA storage encoding method, comprising:
1) obtaining the DNA storage encoding/decoding rule generated by the method of any one of claims 1-8, and setting an initial node as the current node;
2) obtaining a binary sequence to be encoded and slicing it to generate binary slices, and converting the binary values corresponding to the slices into the out-degree node or the multi-layer out-degree node connected with the current node, wherein each out-degree node denotes a nucleic acid fragment and the binary slices form a one-to-one mapping relationship with the corresponding nucleic acid fragments;
3) according to the DNA storage encoding/decoding rule, inputting the binary slices to output the nucleic acid fragment that the out-degree node or the multi-layer out-degree node is mapped to, updating the out-degree node as the current node, and cyclically inputting the binary slices and outputting the nucleic acid fragments according to the order of the binary slices, until all of the binary slices have been input; and
4) connecting the nucleic acid fragments in the output order and outputting the complete DNA sequence.

12. The method of claim 11, wherein the binary sequence to be encoded is sliced with the length of 2n-1, wherein n represents the length of base characters of each sliding of the sliding windows.

13. The method of claim 11 or 12, further comprising: synthesizing the DNA sequence and preserving it in an *ex vivo* medium or in living cells.

14. A DNA storage encoding apparatus, comprising:
a unit for acquiring encoding/decoding rule, configured to obtain the DNA storage encoding/decoding rule generated by the method of any one of claims 1-8, and set an initial node as the current node;
a unit for slicing and converting binary sequences, configured to obtain a binary sequence to be encoded and slice it to generate binary slices, and convert the binary values corresponding to the slices into the out-degree node or the multi-layer out-degree node connected with the current node, wherein each out-degree node denotes a nucleic acid fragment and the binary slices form a one-to-one mapping relationship with the corresponding nucleic acid fragments;
a unit for outputting nucleic acid fragment, configured to, according to the DNA storage encoding/decoding rule, input the binary slices to output the nucleic acid fragment that the out-degree node or the multi-layer out-degree node is mapped to, update the out-degree node as the current node, and cyclically input the binary slices and output the nucleic acid fragments, according to the order of the binary slices, until all of the binary slices have been input; and
a unit for connecting nucleic acid fragments, configured to connect the nucleic acid fragments in the output order and output the complete DNA sequence.

15. The apparatus of claim 14, wherein the binary sequence to be encoded is sliced with the length of 2n-1, wherein n represents the length of base characters of each sliding of the sliding windows.

16. A DNA storage decoding method, comprising:
1) obtaining the DNA storage encoding/decoding rule generated by the method of any one of claims 1-8, and setting an initial node as the current node;
2) obtaining a single-molecule sequenced DNA sequence to be decoded and slicing it to generate nucleic acid slices, and determining the out-degree node or the multi-layer out-degree node connected with the current node according to the DNA storage encoding/decoding rule and the nucleic acid information corresponding to the slices, wherein each out-degree node denotes the information of a nucleic acid fragment and the nucleic acid slices form a one-to-one mapping relationship with the corresponding binary values or binary slices;
3) for the current node and the out-degree node or the multi-layer out-degree node, obtaining the binary value or the binary slice between the nodes according to the mapping, updating the out-degree node as the current node, and cyclically inputting the nucleic acid slices and outputting the binary values or the binary slices, according to the order of the nucleic acid slices, until all of the nucleic acid slices have been input; and
4) connecting the binary values or the binary slices in the output order and outputting the complete binary sequence.

17. The method of claim 16, wherein step 3) comprises an error correction step that uses a path-based probabilistic error correction strategy, comprising:
(1) detecting errors during decoding:
detecting an error according to the constraint, that is, the current node has no access to the next node or does not meet the requirements of a valid path;
(2) determining the position where the error occurs during decoding:
checking in reverse order, *i.e.,* backwards from the position where the error occurs to the position that is the length of an observation window away from the error position;
(3) obtaining candidate repaired sequences:
after finding the position where the error occurs during decoding, performing a local exhaustive backward search to determine the error type and attempt to repair by the following three types of adjustments: replacing the nucleotide at the current position with another nucleotide, inserting a nucleotide between the current position and the previous position, and deleting the nucleotide at the current position, so as to obtain candidate repaired sequences; and
(4) obtaining a set of repaired DNA sequences:
for the candidate repaired sequences, performing path detection based on Varshamov-Tenengolts error correction code to collect the candidate repaired sequences that pass the constraint and meet the requirements of the detected sequence; and outputting a set of repaired DNA sequences that pass the constraint and meet the requirements of the detected sequence.

18. The method of claim 17, wherein after step (4), all of the sequences in the set of DNA sequences are counted and sorted, and the sequences with a frequency reaching a threshold are correct sequences, and directly decoded.

19. The method of claim 17 or 18, wherein the sequencing depth of the sequences is 20×, and the sequence frequency threshold is 4, that is, the sequences with a frequency reaching 4 are correct sequences, and are directly decoded.

20. The method of any one of claims 16-19, wherein the DNA sequence to be decoded is sliced with the length of n, wherein n represents the length of base characters of each sliding of the sliding windows.

21. The method of any one of claims 16-20, wherein the DNA sequence to be decoded is generated by being encoded by the method of any one of claims 11-14 or the apparatus of claim 14 or 15.

22. A DNA storage decoding apparatus, comprising:
a unit for acquiring encoding/decoding rule, configured to obtain the DNA storage encoding/decoding rule generated by the method of any one of claims 1-8, and set an initial node as the current node;
a unit for slicing and converting DNA, configured to obtain a single-molecule sequenced DNA sequence to be decoded and slice it to generate nucleic acid slices, and determine the out-degree node or the multi-layer out-degree node connected with the current node according to the DNA storage encoding/decoding rule and the nucleic acid information corresponding to the slices, wherein each out-degree node denotes a nucleic acid fragment and the nucleic acid slices form a one-to-one mapping relationship with the corresponding binary values or binary slices;
a unit for outputting binary value, configured to, for the current node and the out-degree node or the multi-layer out-degree node, obtain the binary value or the binary slice between the nodes according to the mapping, update the out-degree node as the current node, and cyclically input the nucleic acid slices and output the binary values or the binary slices, according to the order of the nucleic acid slices, until all of the nucleic acid slices have been input; and
a unit for connecting binary value, configured to connect the binary values in the output order and output the complete binary sequence.

23. The apparatus of claim 22, the DNA sequence to be decoded is sliced with the length of n, wherein n represents the length of base characters of each sliding of the sliding windows.
